# EUROPÄISCHE PATENTSCHRIFT

(11) **EP 0 822 436 B1**
(45) Veröffentlichungstag und Bekanntmachung des Hinweises auf die Patenterteilung: **22.01.2003**
(21) Anmeldenummer: 97119033.5
(22) Anmeldetag: 09.04.1995
(51) Int. Cl.: G02B 21/00, G01B 9/04

(54) **Verfahren zur Ermittlung von Positionsdaten und Vorrichtung für das Messen der Vergrösserung in einem optischen Strahlengang**
Method for determining measurement-point position data and device for measuring the magnification of an optical beam path
Procédé permettant de déterminer les données de position de mesure et dispositif permettant de mesurer le grossissement du trajet optique d'un faisceau lumineux

(30) Priorität: 11.04.1994 CH 108894; 11.04.1994 CH 108994; 11.04.1994 CH 109094; 11.04.1994 CH 109194; 11.04.1994 CH 109294
(43) Veröffentlichungstag der Anmeldung: 04.02.1998
(62) Teilanmeldung aus: 95914354.6
(73) Patentinhaber: Leica Microsystems (Schweiz) AG, 9435 Heerbrugg (CH)
(72) Erfinder: Spink, Roger, 9442 Berneck (CH); Braunecker, Bernhard, 9445 Rebstein (CH); Mayer, Thomas, 6845 Hohenems (AT); Zimmer, Klaus-Peter, 9435 Heerbrugg (CH); Rogers, John Rice, Sierra Madre, CA 91024 (US)
(74) Vertreter: Stamer, Harald

(56) Entgegenhaltungen:
- EP-A- 0 164 680
- EP-A- 0 456 103
- WO-A-88/07312
- WO-A-93/16631
- DE-A- 4 134 481
- DE-A- 4 202 505
- NL-A- 9 000 622
- US-A- 4 122 518
- US-A- 4 638 471
- US-A- 4 672 559
- US-A- 4 686 639
- US-A- 5 098 426
- US-A- 5 108 174
- US-A- 5 249 581
- US-A- 5 279 309

## Beschreibung

Die Erfindung betrifft ein Verfahren zur Ermittlung von Positionsdaten eines Messpunktes und eine Vorrichtung für das Messen der Vergrösserung in einem optischen Strahlengang, insbesondere in einem Strahlengang eines Operationsmikroskopes.

Operationsmikroskope dienen einem Operateur zur optischen Vergrösserung des Gebietes, in dem eine Operation durchgeführt werden soll. Es gibt grundsätzlich drei verschiedene Arten von Operationsmikroskopen, die alle im Sinne der Erfindung gemeint sind. Das sind erstens
- reine optische Mikroskope, das heisst, Mikroskope, die nur optische und mechanische Bauteile enthalten, wobei dessen Ausgang dem Auge zugewandt ist; zweitens
- reine Videomikroskope, dass heisst, Mikroskope, die optische, mechanische und optoelektronische Bauteile aufweisen, wobei der optische Ausgang des Mikroskopes ausschliesslich einer optoelektronischen Bildaufnahmevorrichtung (z.B. einem CCD) zugewandt ist und das aufgenommene Bild ausschliesslich elektronisch weiter verarbeitet und gegebenenfalls über ein Display dargestellt wird; und drittens
- gemischte Videomikroskope, die bauliche Merkmale der Mikroskope nach erstens und zweitens gemeinsam enthalten, dass heisst, dass ein Ausgang sowohl einem Betrachterauge als auch einer Bildaufnahmevorrichtung zugewandt ist.

Bedingt durch die Vergrösserung des zu operierenden Gebietes verliert ein Operateur die unmittelbare Grösseneinschätzung, die er bei Operationen mit freiem Auge hat. Dies führt vor allem dort zu Problemen, wo bestimmte, vorher ermittelte Schnittiefen oder Schnittlängen einzuhalten sind, bzw. wo sich der Operateur mit einem Operationswerkzeug an bestimmte Weglängen zu halten hat, um eine präzise Operation zu ermöglichen. Vor allem bei Operationen am Gehirn bzw. bei der Mikrochirurgie ist dies häufig unerlässlich, um Beschädigungen von gesundem Gewebe zu vermeiden. Bei solchen Operationen hängt das Operationsergebnis (ob voller Erfolg oder Exitus) häufig von Bruchteilen von Millimetern ab. Deshalb wurden Anstrengungen unternommen, die Gebiete möglichst genau zu bestimmen und Grössenmessungen zu erlauben. Als Beispiel eines solchen bekannten Aufbaus wird auf die deutsche Patentanmeldung DE-A-4134481 verwiesen.

In der erwähnten DE-A ist ein Operationsmikroskop beschrieben, bei dem eine genaue Ortsbestimmung eines bestimmten, mittels Laserstrahl erzeugten Punktes auf einem betrachteten Objekt erfolgen soll. Dazu ist ein Anvisierverfahren vorgeschlagen, bei dem durch Fokussieren bzw. Defokussieren des Mikroskopes Sehfeldmarkierungen zur Deckung gebracht werden. Danach soll die exakte Bestimmung der Position des Punktes am Objekt möglich sein, indem optische Systemdaten zur Berechnung herangezogen werden. Diese Systemdaten sollen gemäss der DE-A durch geeignete Weg- bzw. Winkeldetektoren an Antriebseinheiten für die jeweilige Verstellung verstellbarer optischer Bauteile ermittelt werden. Namentlich soll daraus auf die Vergrösserung des Vergrösserungssystems geschlossen werden.

Die Ermittlung der Vergrösserung erfolgt somit indirekt über das Messen von Wegen, Winkeln usw. über Sensoren, die mit Verstelleinrichtungen für optische Bauteile verbunden sind und über ein anschliessendes Berechnen der entsprechenden Daten.

Dies ist in vielen Fällen unbefriedigend und ungenügend. Der Hauptgrund liegt darin, dass sowohl die optomechanischen Bauteile als auch die mechanisch/elektrischen Bauteile (Sensoren) über Toleranzen verfügen, die sich u.U. nichtlinear ändern. Daraus resultiert die Gefahr, dass derart ermittelte Vergrösserungswerte falsch sind und daher auch die daraus weiter ermittelten Positionsdaten nicht stimmen. Im Extremfall könnten solche unrichtigen Daten zu folgenschweren Fehlern bei der Arbeit des Operateurs führen. Etwas abgeschwächt werden solche Fehler eventuell durch - gemäss DE-A zwingend vorgesehene - Eichmessungen am Patient. Gerade diese sind jedoch nicht unbestritten und vor allem von der menschlichen Leistung der Bedienperson abhängig. Der bekannte Versuch, mechanische Toleranzen des Vergrösserungssystems bei der Montage des Mikroskopes zu erfassen und daraus eine Korrekturkurve zu ermitteln, die den aktuellen Daten überlagert wird, ist insofern ungenügend, als Toleranzen sich in Abhängigkeit unzähliger Faktoren ändern können und die dann verwendeten Korrekturkurven keinerlei Hilfe sind. Ausserdem ist das Ermitteln solcher Korrekturkurven selbst problematisch, vor allem zeitaufwendig. Ein entsprechendes Korrekturprogramm benötigt darüber hinaus zusätzliche Rechnerleistung und reduziert gegebenenfalls die Rechnergeschwindigkeit im Realtime-Bereich.

Der Erfindung liegt daher die Aufgabe zugrunde, ein Verfahren zu entwickeln, bei dem die erwähnten Nachteile vermieden werden und Positionsdaten schnell und zuverlässig bestimmt werden können. Die Lösung dieser Aufgabe ergibt sich beispielsweise daraus, dass bei der Ermittlung von Positionsdaten die Vergrösserung des Mikroskopes jeweils unmittelbar und unter Vermeidung von mechanischen Elementen bzw. mechanischen Sensoren gemessen wird, wie es in Anspruch 1 dargestellt ist. Der gemessene Wert kann dann beispielsweise im Rahmen bekannter Systeme bzw. Verfahren, z.B. gemäss der erwähnten DE-A, anstelle des dort über Umwegen ermittelten Wertes zur Berechnung der Positionsdaten herangezogen werden. Abgesehen davon kann ein solcher, tatsächlich gemessener Vergrösserungswert auch unmittelbar direkt bei der - z.B. elektronischen bzw. softwareunterstützten - Vergrösserungsänderung allfälliger anderer Bilddaten, wie z.B. MRI- oder Röntgenbilddaten, verwendet werden, sollten solche Bilddaten den aus dem Mikroskop ermittelten Bilddaten mithilfe eines bekannten oder neuen Systems überlagert werden.

Hinsichtlich des Verfahrens zur Bestimmung von Positionsdaten wird insbesondere auf die Beschreibungsteile der erwähnten DE-A- verwiesen. Es sind dies insbesondere: Spalte 2 Zeile 13 bis Spalte 4 Zeile 5 sowie die Figuren 2-4 und die dazugehörigen Beschreibungsteile. Hinsichtlich der Möglichkeit, Bilddaten zu überlagern wird ausserdem auf die folgenden PCT-Patentanmeldungen verwiesen. Es sind dies die Anmeldungen:
WO-A-95 18 511, WO-A-95 18 512, WO-A-95 27 226.

Weiters liegt der Erfindung darüber hinaus die etwas allgemeinere Aufgabe zugrunde, eine Vorrichtung zu schaffen, mit der die Vergrösserung (positive oder negative) in einem Strahlengang mit optischen Bauelementen, insbesondere in einem Mikroskop, unmittelbar gemessen werden kann. Die Messdaten sollen für die Berechnung von Positionsdaten oder aber auch lediglich zur Information der Bedienperson dienen.

Diese Aufgabe wird beispielsweise durch die im Anspruch 6 beschriebenen Merkmale gelöst.

Die Anwendung der beschriebenen Merkmale führt zu einem einfachen Aufbau, der die optischen Eigenschaften des Mikroskops kaum beeinträchtigt. Ein Einblendelement kann infolge des geringen Durchmessers eines Messstrahls, der bevorzugt als Laserstrahl ausgebildet ist, sehr klein gebaut sein. Es kann darüber hinaus in unmittelbarer Nähe zum Hauptobjektiv angeordnet sein, so dass es optisch unter der Wahmehmbarkeitsgrenze liegt. Als Messarray kommen bekannte optische Sensoren wie Diodenarrays, CCD-s etc. in Frage, wobei im Prinzip eine lineare Erstreckung des Arrays ausreicht. Als Einblendelement kommen grundsätzlich alle spiegelnden Bauteile in Frage, wie Strahlenteiler, Spiegel, reflektierende Prismenflächen usw.

Gemäss einer besonderen Ausbildung der Erfindung wird der durch den Strahlenteiler für die Messstrahlausblendung hindurchgehende Teil des Messstrahls durch ein schmalbandiges Filter herausgefiltert, oder der Messstrahl in einem Frequenzbereich gewählt, so dass er dem menschlichen Auge verborgen bleibt.

Die Erfindung ist dabei bei allen oben erwähnten Arten von Mikroskopen anwendbar, wobei bei Videomikroskopen der sich an der Bildaufnahmevorrichtung (z.B. am CCD) ergebende Bildpunkt auch elektronisch entfernbar ist. Andererseits könnte die Bildaufnahmevorrichtung selbst das Messarray ersetzen, wenn der sich darauf ergebende Messpunkt des Messstrahls genügend kontrastreich und ohne Störung des Operateurs darstellen - z.B. elektronisch ausgeblendet - lässt.

Die entsprechend einer erfindungsgemässen Weiterbildung reduzierte Messstrahlsendefrequenz reduziert auf anderem Wege die allfällige Belastung eines menschlichen Auges, ohne die Messgenauigkeit zu beeinträchtigen. Bei Bedarf könnte eine solche Vorrichtung auch mit der Bildaufnahmevorrichtung und mit allfälligen Referenzarrays usw. getaktet bzw. synchronisiert sein, um optimale Wirksamkeit bei geringster Störung aufzuweisen. So kann der Messstrahl beispielsweise immer gerade dann abgegeben werden, wenn die Bildaufnahmevorrichtung nicht für den Empfang von Bilddaten bereit ist. Nachdem sich die Vergrösserung - bei fixer Einstellung des Hauptobjektivs - in der Regel nur durch Änderung der Zoomeinstellung ändert, mag es vorteilhaft genügen, wenn der Messstrahl jeweils nur unmittelbar nach einer Einstellungsänderung am Zoom - die durch Abgriff am Stellmotor des Zoom festgestellt werden kann - abgegeben wird.

Die Erfindung ist insbesondere im Zusammenhang mit einem Operationsmikroskop beschrieben. Im weitesten Sinn kann sie jedoch auch sinnvoll mit beliebigen anderen Strahlengängen angewendet werden.

Im Rahmen der Erfindung liegen verschiedene weitere Verfahren, Ausbildungsarten und Varianten dazu, die in den abhängigen Ansprüchen und in der nachfolgenden Figurenbeschreibung gekennzeichnet bzw. beschrieben sind. Darüber hinaus sind dem Fachmann nach Studium dieser Anmeldung sowie der hierin zitierten Dokumente unterschiedliche Kombinationen verschiedenster Merkmale zu hierin nicht unmittelbar beschriebenen Konstruktionen evident, die ebenfalls im Rahmen der Erfindung liegen.

Weitere Details und Ausführungen der Erfindung ergeben sich aus der Zeichnung. Die dort dargestellten Figuren zeigen:
- Fig.1: das Prinzip eines erfindungsgemässen Aufbaus zur Messung der Vergrösserung eines Mikroskopstrahlenganges;
- Fig.2: das Prinzip einer Variante des Aufbaus nach Fig.1 mit einem herkömmlichen Display zur Einspiegelung von Vergrösserungsinformationen und/oder von anderen Bilddaten;
- Fig.3: eine weitere Variante zur Fig.1 mit zwei Messstrahlen;
- Fig.4: das Prinzip eines Aufbaus mit ausschliesslich visueller Vergrösserungsanzeige im optischen Strahlengang eines Mikroskops;
- Fig.5: drei Varianten für die Anordnung der Ein- und Ausblendelemente im Strahlengang eines Mikroskops;
- Fig.6: einen bevorzugten Aufbau, der die erfindungsgemässe Lehre beispielhaft mit der Erfindung gemäss der am selben Tag eingereichten PCT-Patentanmeldung basieren auf der CH1090/94-1 kombiniert;
- Fig.7: ein Detail eines Einblendelements;

Die Figuren werden zusammenhängend beschrieben. Gleiche Bezugszeichen bedeuten gleiche Bauteile. Gleiche Bezugszeichen mit unterschiedlichen Indizes bedeuten ähnliche bzw. funktionsähnliche Bauteile. Die Erfindung ist auf die dargestellten Ausführungsbeispiele nicht eingeschränkt.

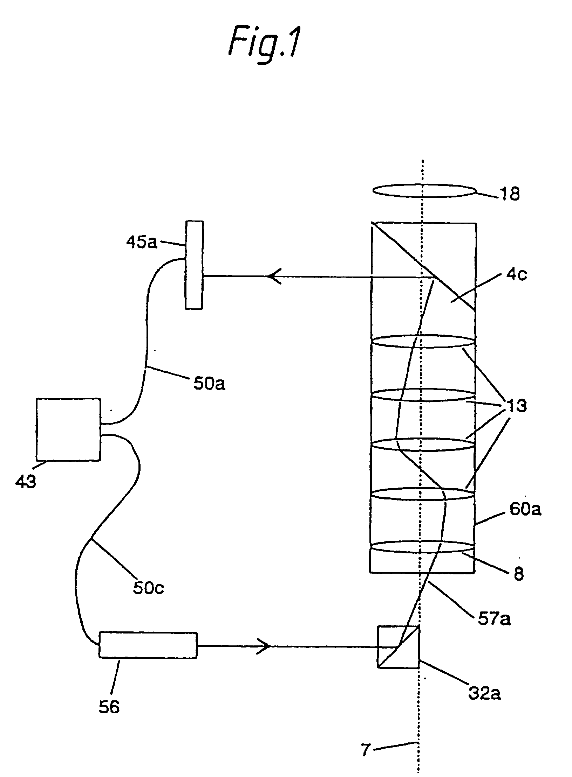

Fig.1 zeigt einen Mikroskopstrahlengang 60a mit einem schematisch angedeuteten Hauptobjektiv 8 und einem Zoom 13. Vor dem Hauptobjektiv 8 ist seitlich der optischen Achse 7 des Strahlenganges 60 ein kleiner Strahlenteiler 32a als Einblendelement justierbar angeordnet. Die Halterung des Strahlenteilers ist nicht dargestellt, da jedem Fachmann eine Vielzahl von geeigneten Halterungen bekannt sind. Der Eingangsseite des Strahlenteilers 32a ist ein Laser 56 zugewandt, der einen Messstrahl 57a auf die Strahlenteilerfläche richtet. Diese ist bevorzugt so ausgerichtet, dass der abgeteilte Strahl 57a unter einem Winkel zur Achse 7 verläuft.

Hinter dem Strahlengang 60a ist ein Strahlenteiler 4c für das Ausblenden des Messstrahls 57a angeordnet. Dessen Ausgang ist einem ortsauflösenden optoelektronischen Messarray 45a zugewandt, an dem der ausgeblendete Strahl 57a aufgefangen wird.

Das erfindungsgemässe Messprinzip liegt darin, dass dem vor dem Hauptobjektiv 8 eingespiegeltem Messstrahl 57a in Abhängigkeit von der Vergrösserung im Strahlengang 60a ein bestimmter Ort auf dem Messarray 45a zukommt, der elektronisch abgefragt und entsprechend ausgewertet werden kann.

Da bei diesem Aufbau der beim Strahlenteiler 4c geradeaus gehende Anteil des Messstrahls 57a durch das Okular 18 in ein Betrachterauge fallen kann, ist hier vorgesehen, dass der Strahl 57a nur fallweise abgesandt wird. Ein Intervallschalter 43, der über die Verbindungsleitung 50a mit dem Messarray 45a und über die Verbindungsleitung 50c mit dem Laser 56 verbunden ist, unterbricht die Strahlenabgabe aus dem Laser 56 fortwährend, so dass nur von Zeit zu Zeit Messungen durchgeführt werden. Dies hat dann Bedeutung, wenn der Messstrahl 57a entweder so hell ist, dass er einen Betrachter stört, oder dass seine Leistung im Infrarotbereich so stark ist, dass am Betrachterauge bei permanenter Einstrahlung Schäden auftreten könnten.

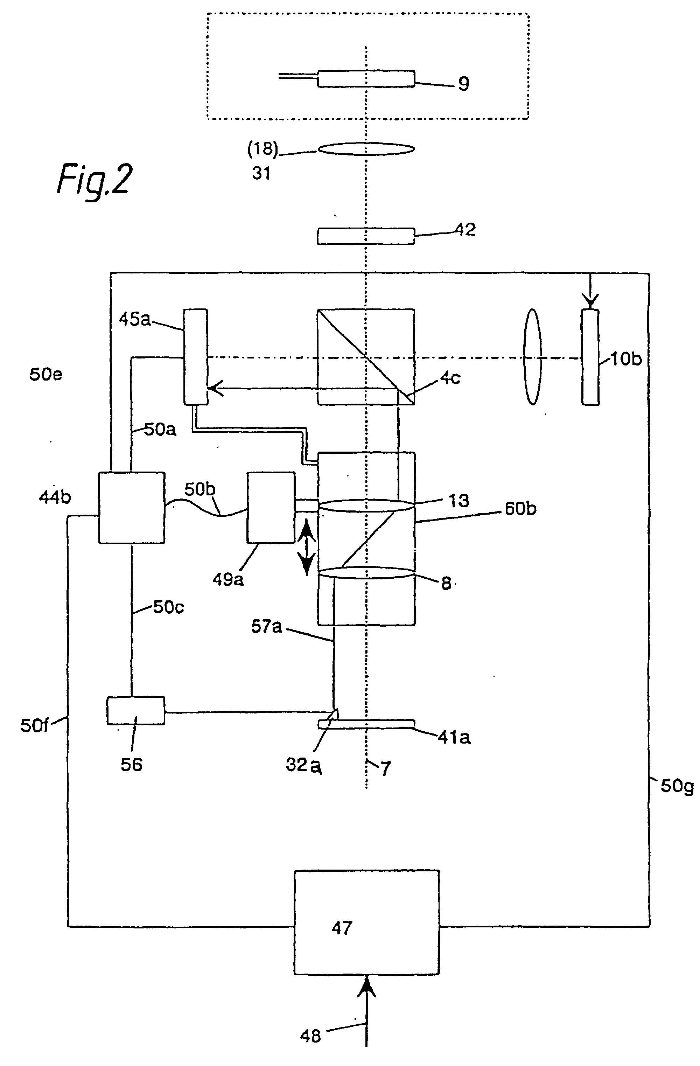

Fig.2 ist insofern unterschiedlich, als zusätzlich zu den beschriebenen Bauteilen ein Stellmotor 49a für das Zoom 13 vorgesehen ist, der über eine Verbindungsleitung 50b mit einem Mikroprozessor 44b verbunden ist. Diese Verbindung ermöglicht Messungen auf ein unbedingtes Mass zu reduzieren, nämlich auf jene Fälle, in denen am Zoom 13 eine Einstellungsänderung vorgenommen wurde. Störungen eines Betrachterauges sind dadurch minimiert.

Durch ein Farbfilter 42, das hinter dem Strahlenteiler 4c angeordnet ist, können Störungen für ein Betrachterauge vollständig eliminiert werden. Das Filter ist bevorzugt sehr schmalbandig und filtert gerade nur die Farbwellenlänge des Messstrahls (die z.B. im Infrarotbereich liegt) heraus.

Das Okular 18 könnte alternativ auch eine Tubuslinse 31 für eine Bildaufnahmevorrichtung 9 sein, die entweder anstelle eines Betrachterauges vorgesehen sein kann oder über bekannte Anordnungen auch zusätzlich dazu - z.B. über einen weiteren Strahlenteiler ausgeblendet - angebracht sein kann.

Bei dieser besonderen Ausgestaltung wird der Strahlenteiler 4c auch als Einblendteil für das Bild eines Displays 10b benutzt, auf dem Bilddaten aus einem Modul 47 für Bilddatenübertragung dargestellt werden können. Das sind in der Regel Röntgenbilddaten, MRI-Bilddaten, oder auch sonstige Information wie z.B. medizinische Werte des Patienten, Positionswerte des Mikroskops oder eben auch Vergrösserungswerte des Mikroskops, die über das Messarray 45a gegebenenfalls nach Umrechnung durch den Mikroprozessor 44b gewonnen werden. Diese Informationen werden dem Display 10b über die Verbindungsleitungen 50e und 50g zugeführt.

Die Verbindungsleitung 50f ermöglicht das Weitergeben der Vergrösserungsdaten an das Modul 47 für Bilddatenübertragung, so dass dort die über den Eingang 48 eingespeisten vorzugsweise genormten Bilddaten aus anderen Systemen Vergrösserungsrichtig umgewandelt an das Display 10b geliefert werden können. Durch diese Einrichtung ist das Überlagern von Bilddaten für einen Operateur besonders komfortabel, da die Dimensionen sicher stimmen.

Das Einblendelement 32a, mit dem der Messstrahl 57a in den Mikroskopstrahlengang 60b eingeführt wird, kann gemäß Fig. 1 als Strahlenteiler ausgebildet sein. Es kann aber auch als Prisma oder als Spiegel gemäß Fig. 2 ausgebildet sein. Das Einblendelement 32a wird in der Regel auf eine Trägerplatte 41a befestigt.

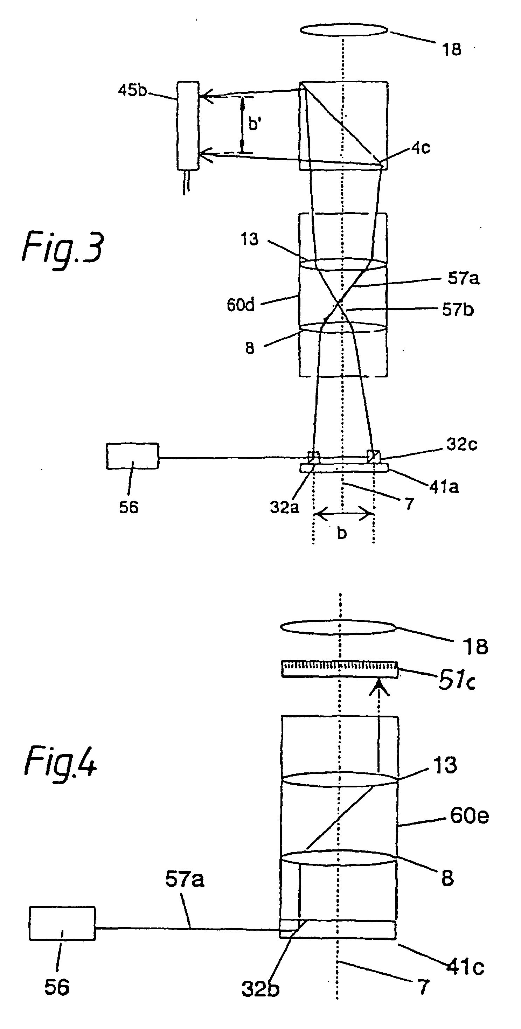

In der Variante gemäss Fig.3 werden zwei Messstrahlen 57a und 57b in bestimmten Winkeln zur optischen Achse 7 durch den Strahlengang 60d gesandt. Dabei ist das Einblendelement 32a als Strahlenteiler ausgebildet, der in Reflexion den Messstrahl 57a und in Transmission und anschließender Reflexion am Einblendelement 32c den Messstrahl 57b erzeugt. Die Relation des am Messarray 45b gemessenen Abstand b' zum bekannten Abstand b entspricht der Vergrösserung des Strahlenganges 60d.

Die spezielle Variante gemäss Fig.4 zeigt einem Betrachter direkt die Vergrösserungswerte, indem dem Strahlengang 60e eine Vergrösserungsanzeige 51c nachgeschaltet ist, an dem eine Skala angebracht ist. Der Betrachter sieht somit durch das Okular 18 nicht nur das beobachtete Bild sondern auch den Messstrahl 57a, der sich bei Vergrösserungsveränderungen an der geeichten Messskala entlangbewegt. Eventuell mag es wünschenswert sein, wenn im Bereich der Messskala die Vergrösserungsanzeige zumindest geringfügig streuend ausgebildet ist.

Als Besonderheit ist in der Darstellung auch ein alternatives Einblendelement 32b dargestellt, das im wesentlichen aus einer gegebenenfalls prismatischen Trägerplatte 41c besteht, in der eine Spiegelfläche 32b integriert ist. Die Trägerplatte 41c ist hier vollflächig dargestellt, es genügt jedoch auch u.U. ein stabförmigen Teil, der nur ein kurzes Stück in den Strahlengang 60e ragt und dadurch zu einer minimalen Beeinträchtigung führt.

Im Sinne der Erfindung ist es nicht wesentlich, ob die angegebenen Bauelemente in genau der Reihenfolge angeordnet sind wie jeweils dargestellt. Es sind verschiedene Variationen denkbar, die entsprechend der gewünschten Bauform Vorteile bringen können.

Alternativ zu den beschriebenen Laserstrahlen sind auch andere gebündelte Lichtstrahlen denkbar, sofern diese die selben Effekte hinsichtlich der Bündelung aufweisen.

Nicht dargestellt sind allfällige Filter, die unmittelbar vor dem Array 45 angeordnet sind, um dort gegebenenfalls störendes Licht aus dem Strahlengang 60 auszublenden.

Der Strahlenteiler 4c könnte entsprechend einer weiteren speziellen Ausgestaltung auch als Strahlenteiler mit einer Strahlenteilerfläche im Brewsterwinkel ausgebildet sein, um einen entsprechend polarisierten Messstrahl 57 vollständig auszublenden und vom Okular 18 fernzuhalten. Ein solcher Aufbau ist dort bevorzugt, wo hohe Lichtstärken am Messarray 45 erforderlich sind.

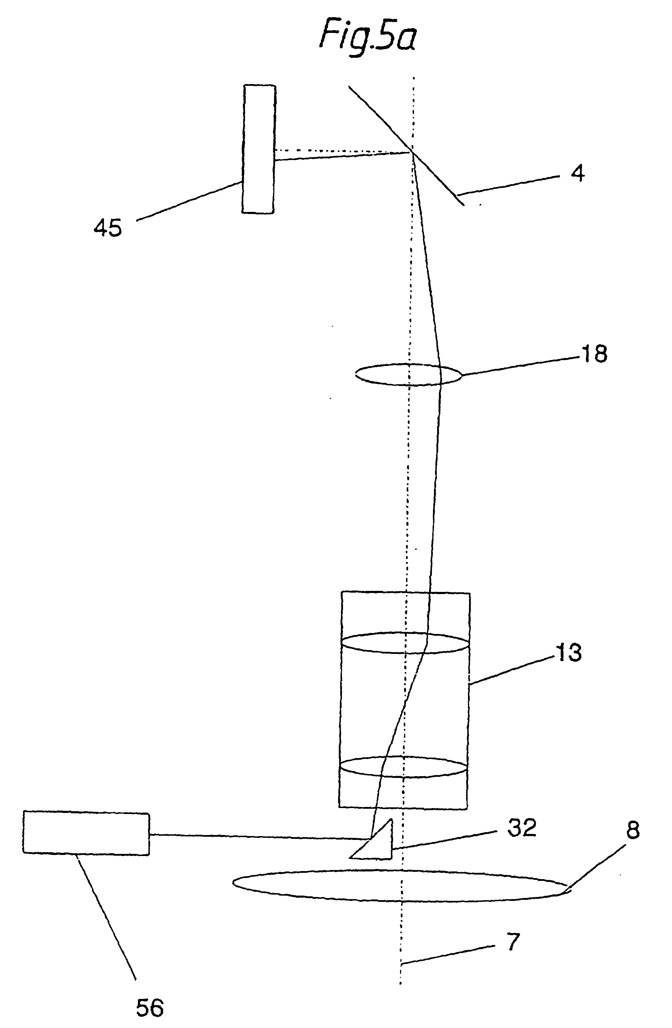

Fig.5a zeigt die Einblendung des Messstrahls hinter dem Hauptobjektiv 8 über ein Prisma 32 unter einem Winkel zur optischen Achse 7. In Abhängigkeit von Winkel und Vergrößerung trifft der Messstrahl nach Auskopplung durch Element 4 auf unterschiedliche Orte des Sensors 45.

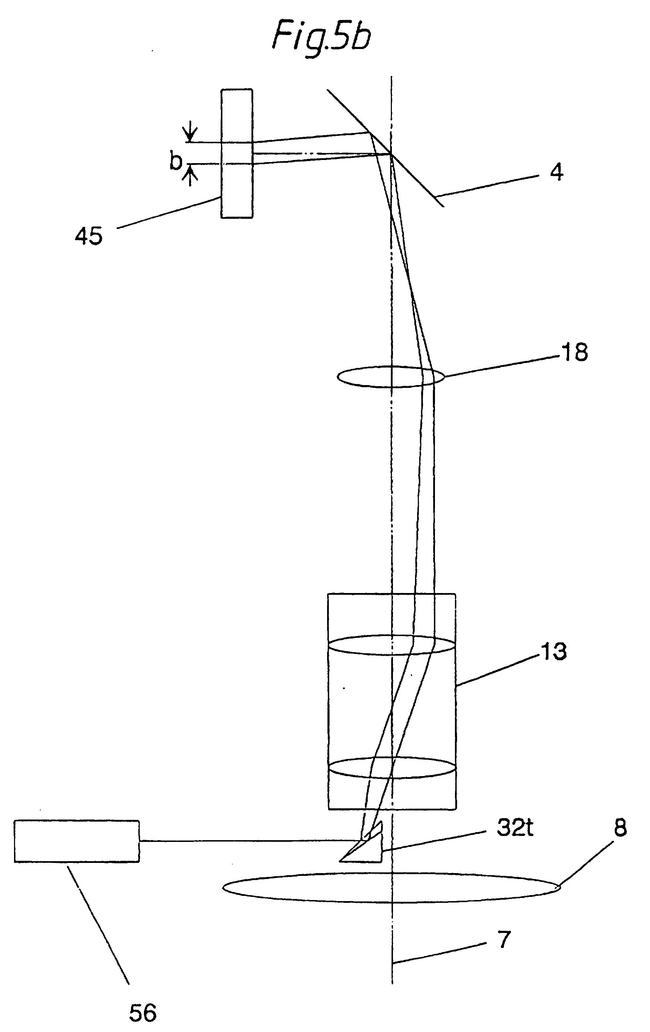

Nach Fig. 5b werden zur Vermeidung von Justierproblemen von Einblendelement 32t zwei Messstrahlen erzeugt, z.B. über zwei in einem definierten Winkel zueinander angeordneten Strahlenteilerflächen, die zwei örtlich getrennte Signale auf dem Sensor 45 erzeugen.

Der Abstand b ist bei bekanntem Winkel ein Mass für die Vergrösserung des optischen Systems. Dieses Prinzip kann auch das Hauptobjektiv zur Bestimmung der Gesamtvergrösserung beinhalten, wenn die Einkopplung vor dem Hauptobjektiv 8 erfolgt.

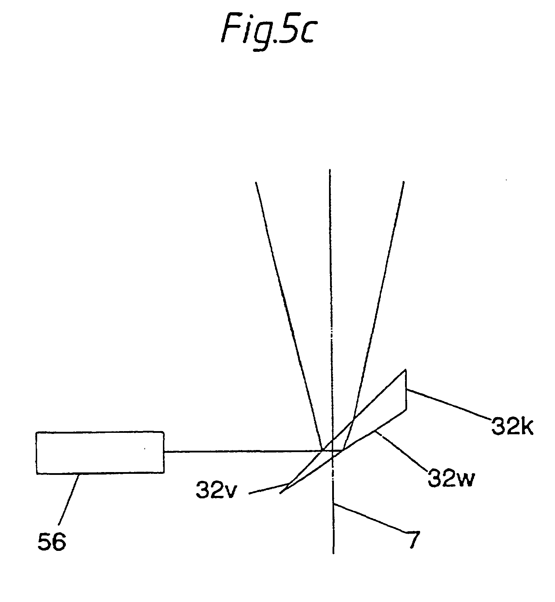

Fig.5c zeigt als weiteres Ausführungsbeispiel eines Einblendelements einen Keil 32k mit den Strahlenteilerflächen 32v und 32w, z.B. mit jeweils 4% Reflektionsgrad. In diesem Ausführungsbeispiel sind die Strahlenverläufe so gewählt, dass sie nach den Reflektionen am Keil 32k symmetrisch zur optischen Achse 7 liegen.

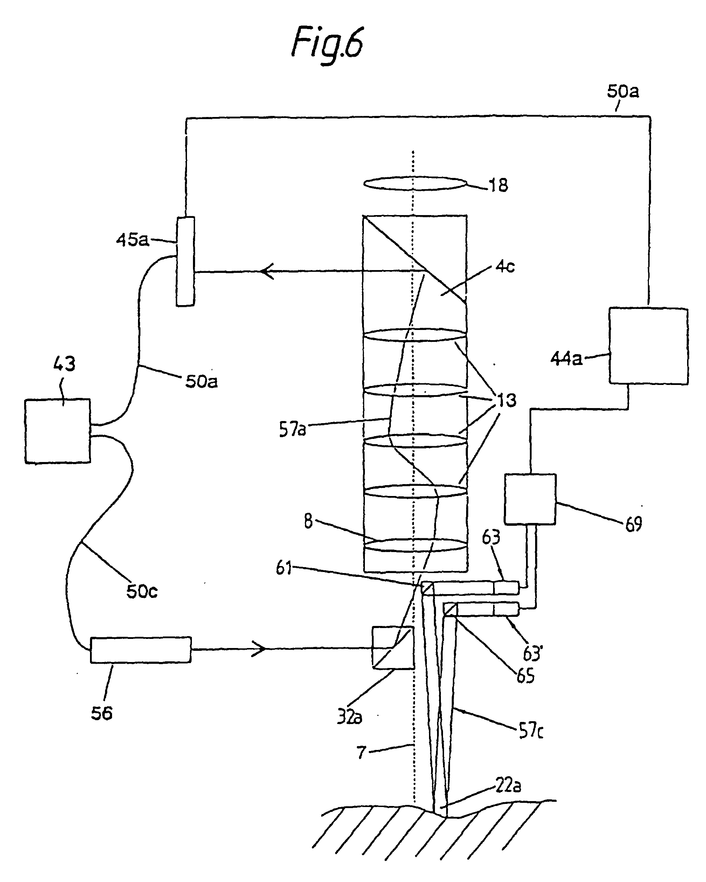

Fig.6 zeigt den Laser 56, dessen Strahl 57a über den justierbaren Strahlenteiler 32a in die Mikroskopoptik 8, 13 umgelenkt wird. Aus der Mikroskopoptik 8, 13 gelangt der Strahl 57a über den Strahlenteiler 4c auf das Messarray 45a. Zum Bestimmen der Vergrösserung, bzw. der Lage der Fokalebene, werden die Strahlenpositionen auf dem Messarray 45a verwendet. Um mögliche vom Messstrahl ausgehende Störungen zu minimieren wird der Laser 56 über den oben bereits erwähnten Intervallschalter 43 gesteuert. Die Auswertung der Positionsdaten erfolgt in einem Mikroprozessor 44a. Die oben beschriebenen Komponenten werden durch Verbindungsleitungen 50 a,c und d miteinander verbunden.

Zusätzlich erfolgt eine Distanzbestimmung über ein Distanz-Messsystem 69, von dem Lichtleiter zu den Endstücken 63 und 63' führen. Es wird Laserlicht verwendet, dessen Stahl 57c zwischen dem Objekt und dem Mikroskop gegen das erste Umlenkelement 61 eingespiesen wird. Der am Objektdetail 22a reflektierte Strahl wird vom zweiten Umlenkelement 65 gegen das mit dem Sensor verbundene Lichtleiter-Endstück 63' umgelenkt. Das Distanz-Messsystem 69 ist mit dem Prozessor 44a verbunden, so dass dieser aus den Distanzwerten und den Vergrösserungswerten auf dem untersuchten Bildausschnitt reale Positionen bestimmen kann.

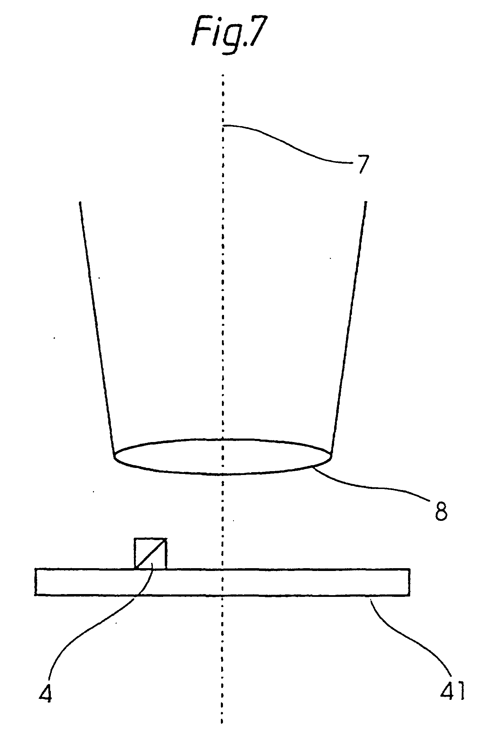

Wie bereits bei den Fig.2, 3 und 4 angedeutet, betrifft die Erfindung auch eine - gegebenenfalls unabhängig einsetzbare - Vorrichtung für das Anordnen von Einblend- bzw. Einspiegel- bzw. Ausblend- und Ausspiegelelementen in einem optischen Strahlengang gemäss Fig.7. Im Bereich der Fotografie und der Mikroskopie gibt es häufig Probleme mit dem Befestigen von optischen Bauteilen für das Ein- oder Ausspiegeln von bestimmten Informationen in einem optischen Strahlengang. Im Bereich der Mikroskopie sind es dabei neuere Entwicklungen, bei denen Laserstrahlen o.dgl. durch den Strahlengang gesandt werden müssen.

Bekannt sind hierzu Strahlenteiler, die in den Strahlengang eingesetzt werden und beispielhaft in den Fig.1, 5 und 6 dargestellt sind. Diese vergrössern jedoch stark das Bauvolumen und schlucken unerwünscht Licht.

Diese Probleme sind nicht zufriedenstellend gelöst. Dem Aspekt der bevorzugten Ausbildung liegt daher die Aufgabe zugrunde, ein Vorrichtung zu schaffen, die unauffällig und ohne den Strahlengang stark zu stören, das Aus- und Einblenden kleiner Strahlenbündel ermöglicht.

Gelöst wird diese Aufgabe durch das Verwenden von den Merkmalen des Patentanspruches 14.

Besondere Ausführungsformen der Erfindung sind in den abhängigen Patentansprüchen 15-16 beschrieben bzw. unter Schutz gestellt. Sie erleichtern vor allem das Reduzieren der Baugrösse von Operationsmikroskopen.

Eine solche Anordnung führt somit zu kompakteren Strahlengängen bzw. Mikroskopen.

Im Rahmen der Erfindung liegen darüber hinaus verschiedene Ausbildungsarten und Varianten dazu, die sich aus der Kombination der hier erwähnten Merkmale mit Merkmalen aus folgenden Patentanmeldungen ergeben, die andere Aspekte eines erfinderischen neuen Mikroskopes unter Schutz stellen, das bevorzugt eben auch mit der vorliegend beschriebenen Anordnung ausgerüstet ist. Es sind dies die bereits erwähnten Patentanmeldungen. Vor allem für den Einsatz zur Vergrösserungs- und Entfernungsmessung ist dieses Detail vorteilhaft.

Weitere Details und Ausführungen der Erfindung ergeben sich aus der Fig.7.

Eine dünne Glasplatte, eventuell antireflexbeschichtet, trägt ein oder mehrere kleine Einblendelemente, die derart knapp an Linsen, Hauptobjektive etc. herangeschoben werden können und nur mehr partielle - in der Regel vernachlässigbar kleine - Störungen verursachen.

Eine bevorzugte Ausbildung der erfindungsgemässen Vorrichtung beinhaltet somit eine Vorrichtung für das störungsfreie Einblenden schmaler Strahlenbündel in optische Strahlengänge. Dazu werden optische Einblendelemente 4 an dünnen Glasscheiben 41 befestigt, die unmittelbar vor Linsen 8 in den Strahlengang eingesetzt sind.

### Bezugszeichenliste

Diese Bezugszeichenliste enthält auch Bezugszeichen von Figuren, die in den oben erwähnten Anmeldungen beinhaltet sind, da diese, bzw. die durch diese Bezugszeichen angeführten Merkmale und deren entsprechenden Beschreibungs- und Zeichnungsteile, wie erwähnt als im Rahmen dieser Erfindung liegend zu Kombinationszwecken mitgeoffenbart gelten. Insbesondere betrifft dies die Mikroskope mit speziellen Strahlengängen und Strahlenteilern und die Vorrichtungen zum Messen der Vergrösserung und des Abstandes vom Mikroskop zum Objekt.
- 1: erster Strahlengang; a,b
- 2: zweiter Strahlengang (geometrisch übereinander gelegte erste Strahlengänge); a,b
- 3: mechanooptisches Schaltelement
3a-c undurchlässige und vorzugsweise verspiegelte Blende
3d LCD-Shutter-Element
3e mikromechanische Lamellenspiegelkonstruktion
3f LCD Wechselshutterelement
- 4: Strahlenteiler
4a,b Strahlenteiler
4c Strahlenteiler für Messstrahlausblendung 4c1, 4c2
- 5: Scheibe
5a halbkreisförmige Fläche -
5b Restfläche der Scheibe 5
5c Kreissegmentflächen 5d
- 6: Achse für Scheibe
- 7: Mittelachse
7a,b Mittelachse
- 8: Hauptobjektiv
8a Hauptobjektiv
8b Hauptobjektiv mit 8a vertauschbar (unterschiedliche Brennweiten)
- 9: elektronische Bildaufnahmevorrichtung
- 10: Display
10a Display
- 11: Spiegel; a,b
- 12: Verstelleinrichtung; a-c
- 13: Zoom
- 14: Motor; a,b
- 15: Reziprokantrieb
- 16: Zuleitung
- 17: Lichtquelle
- 18: Okular
- 19: Umlenkspiegel
- 20: Schubstange
- 21: starrer Spiegel
- 22: Objekt
22a Objektdetail
- 23: Planplatte; a-d,a',b'
- 24: Schwenkantrieb
- 25: Gestänge
- 30: Lamellenspiegel von 3e
- 31: Tubuslinse
- 32: Einblendelement
32a Strahlenteiler
32b Spiegel
32c zweites Einblendelement
- 33: Vergrösserungsoptik
- 34: Pfeile
- 35: weiterer Spiegel
- 36: Stellantrieb
- 37: Balken
- 38: Umlenkspiegel; a,b
- 39: Retroprisma
- 40: Ausgleichsgewicht
- 41: Trägerplatte a,b,c: prismatische mit integriertem Spiegel
- 42: Farbfilter; a-f
- 43: Intervallschalter
- 44: Mikroprozessor
- 45: Messarray a
- 46: Referenzarray a
- 47: Modul für Bilddatenübertragung
- 48: Fremdbilddateninput
- 49: Stellmotor für Zoom 13; a,b
- 50: Verbindungsleitungen a-g
- 51: Vergrösserungsanzeige a-c
- 52: Kurvenscheibe
- 53: Kopplung
53a zwischen Stellmotor 49b und Zoom 13 bzw. zwischen 49 und 52
53b zwischen Kurvenscheibe 52 und Vergrösserungsanzeige 51b
- 54: mechanischer Abgriff
- 55: Zeiger; a,b
- 56: Laser
- 57: Messstrahl a-c,c1
- 58: Referenzstrahl
- 59: Pfeile für Verschiebbarkeit des Einblendelementes 32
- 60: Mikroskopstrahlengang a-e
- 61: erstes Umlenkelement a
- 62: Fokussierelement a,b
- 63: Lichtleiterendstück a,b
- 64: Lichtquelle a
- 65: zweites Umlenkelement
- 66: Sensor
- 67: Distanzbereich a
- 68: Verbindungsleitung
- 69: Distanzmesssystem
- 70: Verbindung
- 71: Vergrösserungsmesseinheit
- 72: Positionsbestimmungssystem a,b
- 73: Interferometer
- 74: halbdurchlässiger Spiegel
- 75: Reflektor
- 76: Detektor
- 77: elektromechanisches Verstellelement
- 78: Interferometersteuerung
- 79: Gitter
- 80: Detektor-CCD
- 81: Stufen
- 82: Mikroskop
- 83: Anordnung zur Vergrösserungsmessung des Mikroskopes
- 84: Anordnung zur Entfernungsmessung Objekt/Mikroskop
- 85: Positionsmessystem zur Bestimmung der Absolutlage des Mikroskopes im Raum um daraus nach Kenntnis der Entfernung Objekt/Mikroskop auch auf die Lage des Sehfeldes am Objekt schliessen zu können
- 86: Toolbox für verschiedene Anwenderprogramme
- 87: Befehlsteuerorgan (Computermouse)
- 88: Befehlsteuerorgan zur Bewegungssteuerung des Mikroskopes (z.B. Fussschalter)
- 89: Datenaufbereitungseinheit
- 90: Computer (Workstation)
- 91: Steuerschalter für Mikroskop
- 92: elektromechanische Steuereinheit für Mikroskop (Zoom, Fokus etc.)
- 93: Leuchtdioden; a-c
- 94: Glasfasern; a-c
- 95: Enden der Glasfasern; a-c
- 96: IR-Rezeptoren; a-c
- 97: Mikroskopständer
- 98: Rückkopplung
- 99: Zuleitungen; a-c
- 100: Reflektoren mit spezieller Oberfläche
- b: Abstand der Messstrahlen 57a und 57b
- b': Abstand der Messstrahlen 57a und 57b am Messarray
- d1,2: Stereobasis

## Patentansprüche

1. Verfahren zur Ermittlung der Positionsdaten eines Messpunktes an einem durch ein Mikroskop betrachteten Objekt, bei dem Positionsdaten des Mikroskopes und Daten über die Fokalebene und die Vergrösserungsdaten des Mikroskopes ermittelt werden und daraus anschliessend die Positionsdaten berechnet werden, **dadurch gekennzeichnet, dass** die Vergrösserungsdaten jeweils unmittelbar mit Hilfe wenigstens eines optischen Messstrahls (57a, 57b), der von aussen kommend zumindest in die Zoom-Optik (13) des Mikroskops eingeblendet wird und durch diese hindurchgeht, aus der Ablenkung des Messstrahls (57a, 57b) - und somit unter Vermeidung von mechanischen Weg- oder Winkelgebern - gemessen und in die Rechnung zur Positionsdatenermittlung eingeführt werden.

2. Verfahren nach Anspruch 1, **dadurch gekennzeichnet, dass** die Messung durch optische Ablenkung des Messstrahls (57a) durch die Mikroskopoptik (8,13) erfolgt, wobei der Messstrahl (57a) im sichtbaren Wellenlängenbereich liegt und dem Messstrahl (57a) eine für den Beobachter sichtbare Messskala (51c) zugeordnet ist, an der dieser den Vergrösserungswert direkt ablesen kann.

3. Verfahren nach Anspruch 1 oder 2, **dadurch gekennzeichnet, dass** die Messung durch optische Ablenkung des Messstrahls (57a) durch die Mikroskopoptik (8,13) auf ein mechanisch festgelegtes ortsauflösendes optoelektronisches Messarray (45, 45a, 45b) erfolgt, dessen elektronischen Ausgangswerte der Vergrösserung entsprechen.

4. Verfahren nach einem der vorhergehenden Ansprüche, **dadurch gekennzeichnet, dass** die Messung durch optische Ablenkung von zwei in bestimmten Winkeln zur optischen Achse (7) des Mikroskops befindlichen Messstrahlen (57a,b) durch die Mikroskopoptik (8,13) auf das optoelektronische Messarray (45b) erfolgt, deren Abstand (b) bekannt ist, wobei der Abstand (b') der abgelenkten Strahlen (57a,b) zueinander bzw. die sich daraus ergebenden elektronischen Ausgangswerte der Vergrösserung entsprechen.

5. Vorrichtung für das Messen der Vergrösserung in einem optischen Strahlengang (60) einer Mikroskopoptik (8,13), mit wenigstens einem einen gebündelten Lichtstrahl erzeugenden Bauelement - insbesondere mit einem Laser (56) zur Erzeugung wenigstens eines Messstrahls (57) und wenigstens einer Vergrösserungsanzeige und/oder einem ortsauflösenden optolektronischen Messarray (45, 45a, 45b) zur Durchführung eines Verfahrens nach einem der vorhergehenden Ansprüche, **dadurch gekennzeichnet, dass** dem Bauelement bzw. dem Laser (56) wenigstens ein Einblendelement (32) nachgeschaltet ist, über das der Messstrahl (57) durch die Mikroskopoptik (8,13), lenkbar ist, und dass der Mikroskopoptik (8,13) wenigstens eine geeichte Vergrösserungsanzeige (51c), an der die Vergrösserung ablesbar ist und/oder wenigstens ein Strahlenteiler (4, 4c) nachgeschaltet ist, an dem der Messstrahl (57) abteilbar und dem Messarray (45) zuführbar ist, und wobei die elektronischen Ausgangswerte des Messarrays (45) der Vergrösserung entsprechen.

6. Vorrichtung nach Anspruch 5, **dadurch gekennzeichnet, dass** der Messstrahl (57) durch ein Einblendelement (32a), das als Strahlenteiler ausgebildet ist, in zwei Messstrahlen (57a,b) aufgeteilt wird, die über das Einblendelement (32a) und über ein zweites Einblendelement (32c) in den Strahlengang des Mikroskopes und über den Strahlenteiler (4c) auf das Messarray (45b) einspiegelbar sind, wobei der Abstand (b) der beiden Messstrahlen (57a,b) festgelegt ist und der Abstand (b') der beiden Messstrahlen (57a,b) am Messarray (45b) der Vergrösserung entspricht.

7. Vorrichtung nach Anspruch 5 oder 6, **dadurch gekennzeichnet, dass** das oder die Einblendelement/e (32) an einer flachen, transparenten Trägerplatte (41a) angeordnet oder ausgebildet sind, die parallel zur Hauptobjektivebene vor dem Hauptobjektiv - gegebenenfalls auswechselbar - in den Strahlengang eingebaut ist.

8. Vorrichtung nach einem der Ansprüche 5 bis 7, **dadurch gekennzeichnet, dass** der Messstrahl (57) in einem Laser (56) erzeugbar ist, dessen Wellenlänge im unsichtbaren Bereich liegt und dessen Ausgangsleistung unterhalb einer für das menschlichen Auge schädlichen Stärke liegt, und/oder dass - in Richtung des Messstrahls durch das Hauptobjektiv betrachtet - hinter dem Strahlenteiler (4c) für die Messstrahlausblendung ein Farbfilter (42) angeordnet ist, dessen vorzugsweise sehr schmalbandige Filterwirkung die Farbfrequenz des Messstrahls filtert.

9. Vorrichtung nach Anspruch 8, **dadurch gekennzeichnet, dass** das Farbfilter (42) ein Fabry-Perot-Filter ist.

10. Vorrichtung nach einem der Ansprüche 5 bis 9, **dadurch gekennzeichnet, dass** der Messstrahl (57) intermittierend und/oder mit dem Messarray (45) und gegebenenfalls mit anderen optoelektronischen (z.B. Bildaufnahmevorrichtung (9)) oder elektromechanischen Bauteilen (z.B. Stellmotor (49a) für ein Zoom (13)) des Mikroskopes getaktet abgebbar ist.

11. Vorrichtung nach einem der Ansprüche 5 bis 10, **dadurch gekennzeichnet, dass** das der am Messarray (45a) ermittelte Vergrösserungswert der Mikroskopoptik über ein Display (10b) in für einen Betrachter geeigneter Form rückgekoppelt bzw. dargestellt wird, wobei das Display (10b) vorzugsweise denselben Strahlenteiler (4c) für die Ausblendung des Messstrahls (57a) zum Einblenden des Displaybildes benutzt.

12. Vorrichtung nach einem der Ansprüche 5 bis 11, **dadurch gekennzeichnet, dass** über den Strahlenteiler (4c) für den Messstrahl (57) das Bild eines bzw. des Displays (10b) in den Betrachtungsstrahlengang einblendbar ist, wobei das Display (10b) dazu vorzugsweise mit einem Modul (47) für Bilddatenübertragung gekoppelt ist, der weiter bevorzugt über einen Mikroprozessor (44b) mit dem Messarray (45a) verbunden ist und beispielsweise vergrösserungsentsprechend angepasste Bilddaten aus anderen Bilddatenquellen (48), z.B. MRI- oder Röntgenaufnahmen, zur Verfügung stellt.

13. Vorrichtung nach einem der Ansprüche 5 bis 12, **dadurch gekennzeichnet, dass** wenigstens der Strahlenteiler (4c) für das Ausblenden des Messstrahls (57) als mechanooptisches Schaltelement ausgebildet ist, dessen Schaltzustand von einem transmissiven in einen reflektiven Zustand alterniert, wobei vorzugsweise während des transmissiven Zustand kein Messstrahl abgegeben wird.

14. Vorrichtung für das Ein- oder Ausblenden von kleinen Lichtbündeln in den optischen Strahlengang einer Anordnung nach einem der Ansprüche 5-13, **dadurch gekennzeichnet, dass** kleine optische Einblendelemente (z.B. Strahlenteiler) (4) an einer dünnen Trägerplatte (41) angeordnet oder ausgebildet sind, die in den Strahlengang eingesetzt ist.

15. Vorrichtung nach Anspruch 14, **dadurch gekennzeichnet, dass** die Trägerplatte (41) in der unmittelbare Nähe von Objektiven oder Linsen angeordnet ist.

16. Vorrichtung nach Anspruch 14 oder 15, **dadurch gekennzeichnet, dass** die optischen Einblendelemente (4) im Strahlengang eines Operationsmikroskops angeordnet sind.

## Claims

1. Method for determining measurement-point position data of an object observed through a microscope in which method the position data of the microscope and data about the focal plane as well as the magnification data of the microscope are determined and in which method the position data are calculated therefrom, **characterized in that** the magnification data are directly measured each time from the deviation of the measurement beam (57a, 57b) by means of at least a measurement beam (57a, 57b) which is at least inserted from outside into the zoom optics (13) of the microscope and transverses the zoom optics - and are therefore measured without using mechanical path or angle sensors - said magnification data being introduced into the calculation for determining the position data.

2. Method according to claim 1, **characterized in that** the measurement is realized by means of optical deviation of the measurement beam (57a) through the microscope optics , the measurement beam (57a) being situated in the range of the visible wave length and **characterized in that** a measurement scale, visible for the observer, is associated to the measurement beam, on which scale the magnification value can be directly read.

3. Method according to claim 1 or 2, **characterized in that** the measurement is realized by means of optical deviation of the measurement beam (57a) through the microscope optics (8, 13) on the optoelectronic, mecanically fixed and locally resolving measurement array (45, 45a, 45b), the electronic output values of the said array corresponding to the magnification.

4. Method according to one of the preceding claims, **characterized in that** the measurement is realized by means of optical deviation of two measurement beams (57a, 57b), situated at determined angles with respect to the optical axis (7) of the microscope, through the microscope optics (8, 13) on the optoelectronic measurement array (45b), the distance (b) between the said beams being known ; the distance (b') between the deviation beams (57a, 57b) respectively the electronic output values therefrom corresponding to the magnification.

5. Device for measuring the magnification in an optical beam path of microscope optics (8, 13) comprising, at least, a component generating a bundled light beam , in particular a laser (57) for generating, at least, a measurement beam (57) and at least a magnification display and/or an optoeclectronic locally resolving measurement array (45, 45a, 45b) for realizing a method according to one of the preceding claims, **characterized in that** the insertion element (32) is situated downstream with respect to the component respectively to the laser (56), and the measurement beam (57) being guidable through the microscope optics (8, 13) via the insertion element and **characterized in that** at least a calibrated magnification display (51c) on which you can read the magnification and/or at least a beam splitter (4, 4c) on which the measurement beam (57) can be splitted and guided to the measurement array (45) is situated downstream with respect to the microscope optics (8, 13); the electronic output values of the measurement array (45) corresponding to the magnification.

6. Device according to claim 5, **characterized in that** the measurement beam (57) is separated into two mesurement beams (57a, b) by means of an insertion element (32) realised as a beam splitter, the two measurement beams can be introduced via the insertion element (32a) and via a second insertion element (32c) into the beam path of the microscope and via the beam splitter (4c) on the measurement array, the distance (b) between the two measurement beams (57a, b) being fixed and the distance (b') between the two measurement beams (57a, b) on the measurement array (45) corresponding to the magnification.

7. Device according to claim 5 or 6, **characterized in that** the insertion elements (32) are arranged or realized on a flat, transparent carrier plate (41a) which is eventually echangeably installed in the beam path, the said plate being installed in front of the main objective parallel with respect to the main objective plane.

8. Device according to one of the claims 5 or 6, **characterized in that** the measurement beam (57) can be generated in a laser (56), the wave length of this laser being situated in the visible range and its ouput power being situated below the dangerous power for the human eye and / or **in that** a color filter (42) is arranged - seen through the main objective in the direction of the measurement beam - behind the beam splitter (4c) for splitting off the measurement beam; the color filter presenting preferably a narrowband 0 filter effect and is filtering the color frequency of the measurement beam.

9. Device according to claim 8, **characterized in that** the color filter (42) is a Fabry - Perot - filter.

10. Device according to one of the claims 5 to 9, **characterized in that** the measurement beam (57) can be intermittently emitted and / or in a cadenced manner by means of the measurement array (45) and eventually by means of other optoelectronic (for example image pickup devices (9)) or electromechanical (for example a servo-motor (49a) for a zoom ) elements of the microscope .

11. Device according to one of the claims 5 to 10, **characterized in that** the microscope optics magnification value determined on the measurement array (45a) is coupled back or represented in a form adapted for the observer, the display (10b) using preferably the same beam splitter (4c) for splitting off the measurement beam (57a) for inserting the display image.

12. Device according to one of the claims 5 to 11, **characterized in that** you can insert the image of the display (10b) into the observation beam path by means of the beam splitter (4c) for the measurement beam (57), the display therefor being coupled preferably by means of a module to transmit the image data which module is preferably linked to the measurement array (45a) by means of a microprocessor (44b) and which module provides for example MRI- images or radiographies from other image data sources (45).

13. Device according to one of the claims 5 to 12, **characterized in that** at least the beam splitter (4c) is realized as a mechanooptical control element for splitting off the measurement beam (57); the said element being able to switch between a transmissive and a reflective state, no measurement beam being preferably emitted during the transmissive state.

14. Device for insertion and splitting off of small light bundles in the optical beam path of an arrangement according to one of the claims 5 to 13, **characterized in that** small optical insertion elements (for ex. beam splitters (4)) are arranged or realized on a thin carrier plate (41) which is introduced into the beam path .

15. Device according to claim 14, **characterized in that** the carrier plate (41) is arranged in the direct vicinity of objectives or lenses.

16. Device according to claim 14 or 15, **characterized in that** the optical insertion elements (4) are arranged in the beam path of a surgical microscope.

## Revendications

1. Procédé permettant de déterminer les données de position d'un point de mesure sur un objet observé par un microscope, dans lequel procédé on détermine les données de position du microscope et des données sur le plan focal, des données d'agrandissement du microscope avec lesquelles données on calcule ensuite à partir de ces données les données de position, **caractérisé en ce que** l'on mesure grâce à la déviation du rayon de mesure (57a, 57b), donc en évitant des capteurs mécaniques de trajets et d'angles, les données d'agrandissement à chaque fois directement à l'aide d'au moins un rayon optique de mesure (27a, 57b) qui est inséré à partir de l'extérieur au moins dans l'optique de zoom (13) du microscope et lequel rayon traverse ladite optique et **caractérisé en ce qu'**on introduit lesdites données d'agrandissement dans le calcul pour la détermination des données de position.

2. Procédé selon la revendication 1, **caractérisé en ce qu'**on mesure la déviation optique du rayon de mesure (57a) à travers l'optique (8, 13) du microscope, le rayon de mesure (57a) se trouvant dans la zone des longueurs d'onde visibles et où on associe au rayon de mesure (57a) une échelle de mesure (51c) visible par l'observateur, sur laquelle échelle on peut lire directement la valeur d'agrandissement.

3. Procédé selon la revendication 1 ou 2, **caractérisé en ce qu'**on effectue la mesure à l'aide de la déviation optique du rayon de mesure (57a) à travers l'optique du microscope (8, 13) sur une zone de mesure (45, 45a, 45b) optoélectronique, à résolution locale et fixé mécaniquement dont les valeurs électroniques de sortie correspondent à l'agrandissement.

4. Procédé selon une des revendications précédentes, **caractérisé en ce que** l'on effectue la mesure à l'aide de la déviation optique de deux rayons de mesure (57a, 57b) situés à certains angles par apport à l'axe optique (7) du microscope à travers l'optique du microscope (8, 13) sur la zone de mesure (45, 45a, 45b) optoélectronique ; la distance (b) entre les deux rayons de mesure étant connue ;la distance (b') située entre les rayons déviés (57a, 57b), respectivement les valeurs de sortie résultant de la déviation, correspondant à l'agrandissement.

5. Dispositif pour mesurer l'agrandissement dans un trajet optique de rayon (60) d'un optique de microscope (8, 13) avec au moins un composant - comportant notamment un laser (56) - générant un rayon de lumière en faisceaux pour générer au moins un rayon de mesure (57) et comportant au moins un dispositif d'affichage d'agrandissement et / ou une zone de mesure (45, 45a, 45b) à résolution locale pour réaliser un procédé selon une des revendications précédentes, **caractérisé en ce qu'**on a disposé au moins un élément d'insertion (32) en aval du composant ou du laser, via lequel élément d'insertion, le rayon de mesure (57) peut être guidé à travers l'optique du microscope (8, 13) et ce qu'on a disposé en aval de l'optique de microscope (8, 13) au moins un affichage d'agrandissement calibré (51c) sur lequel on peut lire l'agrandissement, et au moins un diviseur de rayon (4, 4a) sur lequel on peut diviser le rayon de mesure (57) et le guider vers la zone de mesure (45) ; les valeurs électronique de sortie de la zone de mesure (45) correspondant à l'agrandissement.

6. Dispositif selon la revendication 5, **caractérisé en ce que** le rayon de mesure (57) est divisé au moyen d'un élément d'insertion (32a), qui est réalisé en tant que diviseur de rayon, en deux rayons de mesure (57a, b) qui peuvent être introduits dans le trajet de rayon du microscope via l'élément d'insertion (32a) et via un deuxième élément d'insertion (32c) dans le trajet du rayon du microscope et introduit dans la zone de mesure via le diviseur de rayon (4c), la distance (b) entre les deux rayons de mesure étant déterminée et la distance (b') entre les deux rayons de mesure (57a, 57b) sur la zone de mesure (45b) correspondant à l'agrandissement.

7. Dispositif selon la revendication 5 ou 6, **caractérisé en ce que** le ou les élément (s) (32) d'insertion sont disposés et réalisés sur une plaque (41a) plate et transparente de support qui est installée devant l'objectif principal - éventuellement échangeable - dans le trajet de rayon.

8. Dispositif selon une des revendications 5 à 7, **caractérisé en ce que** le rayon de mesure (57) peut être généré dans un laser (56) dont la longueur d'onde est située dans la zone visible et dont la puissance de sortie se trouve en dessous d'une puissance nuisible pour l'oeil humain et / ou **en ce qu'**un filtre de couleur soit disposé - vu en direction du rayon de mesure à travers l'objectif principal - derrière le diviseur de rayon (4c) pour l'enlèvement du rayon de mesure , où l'effet de filtrage, de préférence à bande étroite , enlève par filtrage la fréquence de couleur du rayon de mesure.

9. Dispositif selon la revendication 8, **caractérisé en ce que** le filtre de couleur (42) est un filtre Fabry - Perrot.

10. Dispositif selon une des revendications 5 à 9, **caractérisé en ce que** le rayon de mesure (57) peut être émis de façon intermittente et / ou de façon cadencée à l'aide de la zone de mesure (45) et éventuellement à l'aide d'autres composants optoélectroniques (par exemple des dispositifs d'enregistrement d'images (9)) ou électromécaniques (par ex, un servomoteur (49a) pour un zoom ) du microscope .

11. Dispositif selon une des revendications 5 à 10, **caractérisé en ce que** la valeur d'agrandissement qui a été déterminée sur la zone de mesure (45a) est reconduite vers l'optique du microscope via un dispositif d'affichage (10b) sous une forme adaptée à l'observateur où la valeur d'agrandissement est représentée sur un dispositif d'affichage, le dispositif d'affichage (10b) utilisant, de préférence, le même diviseur (4c) de rayon pour enlever le rayon de mesure (57a) et pour insérer l'image de l'affichage.

12. Dispositif selon une des revendications 5 à 11, **caractérisé en ce qu'**on peut insérer pour le rayon de mesure (57) via le diviseur de rayon (4c) l'image d'un ou des dispositifs d'affichage (10b) dans le trajet de rayon d'observation , où le dispositif d'affichage (10b) est couplé à cet effet de préférence à un module (47) pour le transfert de données d'image, lequel module est, en outre, lié, de préférence, via un microprocesseur (44b), à la zone de mesure (45a) et fournit en fonction de l'agrandissement des données d'image adaptées, par exemple, à partir d'autres sources de données d'image (48) comme par ex. des images MRI ou des radiographies.

13. Dispositif selon une des revendications 5 à 12, **caractérisé en ce qu'**au moins le diviseur de rayon (4c) est réalisé , pour enlever le rayon de mesure (57), en tant qu'élément de commutation mécano-optique dont 1 'état de commutation alterne d'un état transmissif vers un état réflexif, où l'on émet de préférence aucun rayon de mesure pendant l'état transmissif.

14. Dispositif pour l'insertion ou l'enlèvement de petits faisceaux de lumière dans le trajet du rayon optique d'une disposition selon une des revendications 5 à 13, **caractérisé en ce que** des petits éléments optiques d'insertion (par ex., des diviseurs de rayon ) (4) sont disposés ou réalisés sur une mince plaque de support (41) qui est insérée dans le trajet de rayon.

15. Dispositif selon la revendication 14, **caractérisé en que** la plaque de support (41) est disposée dans le voisinage proche des objectifs ou lentilles .

16. Dispositif selon la revendication 14 ou 15, **caractérisé en ce que** les éléments optiques d'insertion (4) sont disposés dans le trajet de rayon d'un microscope chirurgical.
